# EUROPEAN PATENT APPLICATION

(11) **EP 0 534 043 A2**
(43) Date of publication of application: **31.03.1993**
(21) Application number: 92101068.2
(22) Date of filing: 23.01.1992
(51) Int. Cl.: G01N 33/53, G01N 33/569, G01N 33/564

(54) **Novel antistreptolysin-o reagent for use with Beckman nephelometer system**

(30) Priority: 25.09.1991 US 765629
(71) Applicant: BIOKIT SA, E-08186 Barcelona (ES)
(72) Inventor: Sales-Amill, Miquel, E-08026 Barcelona (ES)
(74) Representative: Cohausz & Florack Patentanwälte

(57) **Abstract**

A reagent for detecting the presence of an antibody to an antigen in a sample comprising the antigen in admixture with Rheumatoid Factor antibody. The reagent is useful in a method for screening for antibody in a sample comprising the steps of:
(a) contacting the sample to be screened for antibody with the reagent, and
(b) detecting an immunoprecipitation from an immunocomplexing of antibody and antigen to produce a signal indicative of the presence of antibody in the sample. The reagent is particularly steptolysine-O in admixture with Rheumatoid Factor antibody and is particularly useful in a method for detecting anti-streptolysine-O.

## Description

### BACKGROUND OF THE INVENTION

### 1. Field of the Invention

Initiation of an immunoreaction between an antigen and its antibody is a common means of detecting the presence of the antibody or the antigen in a sample.

For example, Streptolysine-O (SLO) is a potent antigenic hemolytic toxin produced by group A streptococci. Measurement of the immunoreaction of SLO with its antibody anti-Streptolysine-O (ASO) is used to detect the presence of ASO in a variety of devices. Thus, measurement of ASO titer in serum is used for the diagnosis of such streptococcal infections as rheumatic fever and glomerulonephritis.

The most common devices currently in use for the detection of an antigen-antibody reaction are spectrophotometers or nephelometers, which use optical detection systems. For example, Beckman Instruments markets a nephelometer commonly known as the ARRAY Protein System. The ARRAY system measures the intensity of light as it is scattered by particles in suspension when a beam of light is passed throughout the cell. Such particles are formed by the antigen-antibody immunoprecipitation. The consequent change in the intensity of scattered light occurs at a rate that increases gradually and proceeds through a peak rate of change (peak rate value). The ARRAY system derives the peak rate value and converts this peak rate value into concentration units. Other devices, such as turbidimeters, or manual methods, like slide tests, have also been used to detect antigen-antibody agglutination. In all these devices, the principle used to detect the antigen component in the apparatus is the same, i.e., the specific immunoreaction of the antigen with its antibody.

Use of these devices to detect the antigen-antibody immunoreaction has often proved problematic due to a lack of sensitivity in the system. Accordingly, there is the need to increase the sensitivity of these systems to detect the antigen-antibody immunoreaction.

### 2. Description of Related Art

D. Collet-Cassart et al, "Automated Particle-Counting Immunoassay for Digoxin", Clin Chem, 27 (7), pp. 1205-1209 (1981), and J.C. Mareschal et al., "Homogeneous latex immunoassay for thyroid hormone testing", J. Immuno. Meth., 131, pp. 137-142 (1990), both describe immunoassays based on latex particle technology, the reagents comprising (1) antigen (T₃ or T₄ hormones and Digoxin drug) coated onto a latex particle surface and (2) Rheumatoid Factor (RF) in admixture with an antibody to the antigen coated onto the latex surface. The immunoassays are competition inhibition tests: the antigen (hormone or drug) present in the patient's sample competes for the antibody with the antigen coated onto the latex particle surface.

### SUMMARY OF THE INVENTION

Accordingly, it was one object of the present invention to provide for a method of increasing the sensitivity of known devices to detect the antigen-antibody immunoreaction.

It was another object of the present invention to provide for a method of increasing the sensitivity of known devices to detect the SLO-ASO immunoreaction.

It was another object of the present invention to provide for a reagent which is useful to detect antibody and which helps to amplify the antigen-antibody immunoreaction in devices currently used to detect the antigen-antibody immunoreaction.

It was another object of the present invention to provide for a reagent which is useful to detect ASO and which helps to amplify the SLO-ASO immunoreaction in devices currently used to detect the antigen-antibody immunoreaction.

It was still another object of the present invention to provide a method for screening for streptococcal infections.

These and other objects are met with the present invention, which relates generally to a novel reagent that can be used to amplify an antigen-antibody immunoreaction, for example, the SLO-ASO immunoreaction.

According to the present invention, it has now been discovered that the sensitivity of the system for detecting an antigen-antibody immunoreaction can be increased if the Rheumatoid Factor (RF) antibody is included in the reagent. Specifically, it has been discovered that the presence of Rheumatoid Factor (RF) in the reagent serves to enlarge or enhance the immunoprecipitation reaction between the antigen and the antibody. The IgM class RF reacts with the immunocomplex, thereby increasing the signal produced in the detection device, thereby, making possible the antibody detection.

The incorporation of RF into a reagent for the detection of ASO has proven particularly useful for enlarging or enhancing the immunoprecipitation reaction between SLO and ASO, thereby making possible the ASO detection in a variety of devices.

The present invention also relates to a novel method for screening for antibody in a sample comprising the steps of:
(a) contacting the sample to be screened for antibody with the novel reagent according to the present invention, and
(b) detecting an immunoprecipitation from an immunocomplexing of antibody and antigen to produce a signal indicative of the presence of antibody in the sample.

Particularly, the present invention also relates to a novel method for screening for anti-streptolysine-O in serum comprising the steps of:
(a) contacting the serum to be screened for anti-streptolysine-O with the novel reagent according to the present invention, and
(b) detecting an immunoprecipitation from an immunocomplexing of anti-streptolysine-O and streptolysine-O to produce a signal indicative of the presence of anti-streptolysine-O in the serum.

The novel reagent according to the present invention is useful to detect the antigen-antibody immunoreaction in a variety of devices, such as spectrophotometers, nephelometers, turbidimeters, or any other system of measuring agglutination or precipitation to detect antibodies. The novel reagent according to the present invention has been found to be particularly useful to detect the antigen-antibody immunoreaction in the ARRAY Protein System marketed by BECKMAN Instruments. In this regard, it has also been discovered that the presence of ASO antibodies can be detected in the ARRAY system by their specific precipitation with SLO. The reagent according to the present invention produces immunoprecipitation when ASO antibodies are present in the patient sera, thereby amplifying the signal.

### BRIEF DESCRIPTION OF THE DRAWING

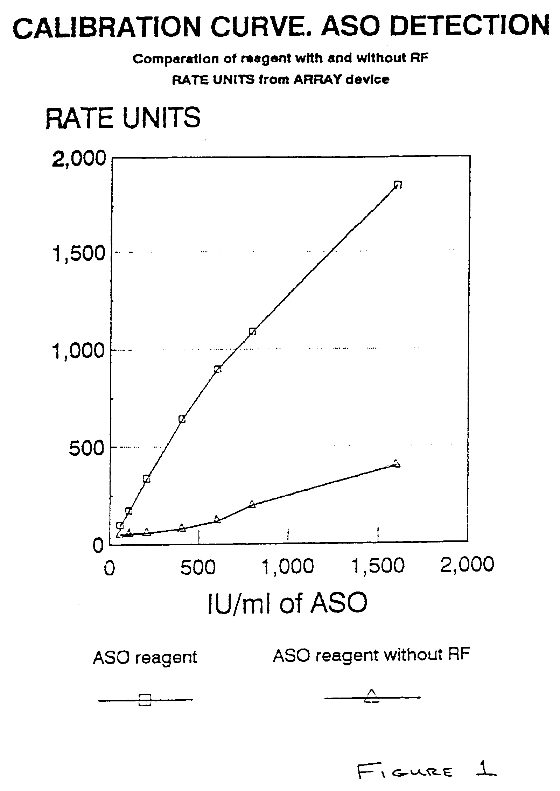

Figure 1 is a graph calibration curve comparing ASO detection using the novel reagent according to the present invention with ASO detection using a reagent without RF.

### DETAILED DESCRIPTION OF THE INVENTION

The novel reagent according to the present invention contains mainly antigen and IgM Rheumatoid Factor (RF). Where it is desired to detect the presence of ASO antibodies in patient sera, the novel reagent according to the present invention will contain mainly SLO antigen and IgM Rheumatoid Factor (RF). The reagent is buffered with phosphate buffer at about pH 7.0: Other additives beside SLO and RF are the BSA to give a protein matrix that could be substituted by another protein like rabbit serum albumin or human serum albumin and also a non-ionic surfactant, such as Tween-20 or other polyoxyethylenes like Brij 3J, Thesit or Tween-80.

The antigen should be purified as much as possible to prevent the possibility of undesirable cross-reactions. In the case of streptococcus detection, SLO is an exotoxin produced from Streptococcus-β hemolytic of the A group. The SLO is purified by adsorption on phosphate gel, followed by precipitation with ammonium sulphate and gel filtration chromatography. The protein is purified as much as possible in order to avoid possible cross-reaction with other anti-exotoxins of the Streptococcus. Purity should be checked after protein purification by specifically testing the activity of the SLO. Purity level is determined as specific activity in International Units/mg (IU/mg) and adequate purity levels are particularly those higher than 1100 IU/mg. Chromatographic and electrophoretic methods may also be used to check the SLO purity. It has been found that SLO purification by adsorption on phosphate gel, followed by precipitation with ammonium sulphate and gel filtration chromatography allows relatively quick processing of thousands of liters of SLO with the requisite purity.

The SLO can, of course, be purified by other methods well known in the art. SLO purification is widely described in the literature, using many purification steps in order to reach a high purity level. For example, purification of SLO is described in Alouf, J.E., "Purification and some properties of SLO", BIOCHIMIE 1973, 55, pages 1187-1193; and Bhakdi, Sucharit, "Isolation and identification of two hemolytic forms of Streptolysin-O", INFECTION AND IMMUNITY, November 1984, pages 394-400.

After purification, it may be necessary to covalently bond the antigen to a bifunctional agent in order to stabilize it. For example, without stabilization, SLO loses more than 50% of its activity, i.e., the ability to react with its specific antibody, after one day at 37°C. Accordingly, in those cases in which the reagent is not to be used immediately, the SLO needs to be stabilized through covalent bonding to a bifunctional agent. Examples of such bifunctional agents are glutaraldehyde and carbodiimide. Preferably, glutaraldehyde serves as the bifunctional agent, giving a protein that is stable for up to a month at 37°C. This bifunctional agent produces inter and intramolecular linkages. Covalent coupling is physically irreversible in the sense that the covalent coupling is not affected by pH or temperature changes.

RF is obtained from human sera and purified by salting out with ammonium sulphate and gel filtration chromatography according to methods well known in the art. The RF used according to the present invention is of the IgM class and purification of IgM class immunoglobulins are well described in the literature. In general, any procedure to generally purify IgM can be used to purify RF according to the present invention. However, purification by salting out with ammonium sulphate and gel filtration chromatography is the preferred procedure. As a example of IgM purification including gel filtration chromatography, but not ammonium sulfate precipitation, see, METHODS IN ENZYMOLOGY, Volume 73, Part B, pages 616-617.

Immunocomplex formation takes place after joining antigen and antibody solutions like SLO and ASO in the detection device. For example, in the case of the ARRAY Protein System, the formation of the immunocomplex in solution increases the turbidity of the solution. Increasing the turbidity increases the scattering of light. The variation in light scattered is plotted versus time (RATE). The analyzer scans for the maximum RATE and this is proportional to the concentration of the protein in the sample being analyzed.

RF of the IgM class has a decavalent activity. The RF acts as a second immunoreaction system after the initial immunoprecipitation of the antigen and antibody to form the immunocomplex. In the case of the ARRAY Protein System, the RF reacts with the immunocomplex, thereby amplifying the light scattered signal in the system.

For the detection of a given antibody, the amount of antigen and RF used will vary somewhat depending on the device. In the case of detection of ASO in the ARRAY system, SLO concentration to detect ASO should be between about 5 and 50 IU/test and RF concentration between about 2 and 30 IU/test. The best SLO and RF concentrations to use in the ARRAY are about 30 IU/test for SLO and about 8 IU/test for RF. The reagent volume is fixed to 42 µm, SLO concentration in the reagent should be about 715 IU/ml and RF concentration about 190 IU/ml.

The invention will now be described in more detail with reference to the following non-limiting examples.

### EXAMPLE 1

### PREPARATION OF SLO

Streptococcus β-hemolytic of group A is grown in a culture media. The culture composition includes yeast extract 25 g/l, peptones 35 g/l, and glucose at 2% concentration in a phosphate 50 mM buffered media. Culture media has been previously heat sterilized at 118°C for 5 minutes. After 18 hours culture at 37°C, the supernatant is removed by centrifugation at 10,000xg. Addition of CaCl₂ at 1% concentration to the supernatant produces a phosphate gel with protein adsorbtion. After two hours, phosphate gel is centrifuged at 10,000xg, proteins are desorbed from phosphate gel by the addition of sodium phosphate 0.2 M. The gel is removed by centrifugation at 10,000xg. Supernatant is then precipitated by the addition of ammonium sulfate to 60% concentration and leaving the precipitate at 4°C overnight. The next day, the protein is centrifuged and the pellet resuspended and dialyzed against PBS (phosphate buffered saline) 10 mM pH 7.0. After complete dialysis, the SLO is passed through a gel filtration chromatography Sephadex G-200 (Pharmacia). The hemolytic pic indicates the presence of purified SLO. The hemolytic pic is then collected and pooled as purified SLO and kept frozen at -20°C.

### EXAMPLE 2

### PREPARATION OF SLO

Streptococcus β-hemolytic of group A is grown in a culture media. The culture composition includes yeast extract, peptones, and glucose in a phosphate 50 mM buffered media in the same concentration as in Example 1. After 10 hours of growing, gel phosphate is developed in the culture by the addition of ZnCl₂ at 0.8% concentration. After two hours, phosphate gel and Steptococcus are centrifuged at 10,000xg, proteins are desorbed from phosphate gel by addition of sodium phosphate 0.2 M. Gel phosphate and Streptococcus are removed by centrifugation at 10,000xg. The supernatant is then precipitated by addition of ammonium sulfate to 60% concentration and leaving the precipitate at 4°C overnight. The next day, the protein is centrifuged and the pellet resuspended and dialyzed against PBS 10 mM pH 7.0. After complete dialysis, the SLO is passed through a gel filtration chromatography Sephadex G-200 (Pharmacia). Elution of fractions is achieved by the use of the same PBS. The hemolytic pic is then collected and pooled as purified SLO and kept frozen at -20°C.

### EXAMPLE 3

### STABILIZATION OF SLO

SLO is brought to room temperature and phosphate buffer is added up to a final concentration between 10 mM and pH 7.0. Glutaraldehyde is then added to a final concentration of 0.1%. The solution is then mechanically agitated at room temperature for 3 hours. Thereafter, glycine is added to a final concentration of 0.1%. The glycine is used to block the rest of the active groups of glutaraldehyde. Covalent coupling is completed by agitation of stabilized SLO at 4°C overnight. The solution is then subjected to extensive dialysis of SLO against phosphate buffer 30 mM pH 7.0 to eliminate the uncoupled glutaraldehyde and glycine. SLO is kept frozen at -20°C.

### EXAMPLE 4

### STABILIZATION OF SLO

SLO is brought to room temperature and Boric buffer is added up to a final concentration of 20 mM and pH 8.5. Glutaraldehyde is then added to a final concentration of 0.02%. Thereafter, the solution is mechanically agitated at room temperature for 3 hours. The solution is then subjected to extensive dialysis of SLO against glycine buffer 50 mM pH 8.2. The SLO is kept frozen at -20°C.

### EXAMPLE 5

### PREPARATION OF RF

Serum samples containing over 1,000 IU/ml of RF are chosen. RF serum is precipitated with ammonium sulfate at 60% after two hours at room temperature. After centrifugation at 5,000xg for 10 minutes, the pellet is resuspended in distilated water and dialized against PBS pH 7.0. Then a gel filtration chromatography column of Superose 6 (Pharmacia) is developed. The same PBS is used to elute protein fractions. Activity is followed by specific titration of RF in a turbidimetric system (Quantex RF kit from BIOKIT SA in the MONARCH autoanalyzer from Instrumentation Laboratory Inc.). Pic activity is collected, pooled and kept frozen at -20°C.

### EXAMPLE 6

### PREPARATION OF THE REAGENT

190 IU/ml of the purified RF and 715 IU/ml of the stabilized SLO dialyzed against PBS 13 mM pH 7.0 are mixed at room temperature with 10 mg/ml BSA and Tween-20 at 1.0 g/l final concentration. 1.0 g/l sodium azide is then added as a preservative. After mixing all of the compounds, the final reagent is filtered through a 0.45 µm filter (Millipore) and kept at 4°C.

### EXAMPLE 7

### USE OF THE REAGENT IN THE ARRAY

A calibration curve is developed using 6-10 serum samples of different ASO concentrations as calibrators, ranging from 100 to 2000 IU/ml of ASO. The reaction buffer and the APO-diluent to dilute all samples are on line in the instrument. Sera dilutions are made manually off-line and use in ARRAY dilution modus "NET", i.e., without dilution. Sera dilutions are made 10 minutes before running the samples. Turbidity from serum samples may disturb the test. If samples are turbid, they must be centrifuged 10 minutes at 5,000xg before testing. Reagent and buffer addition to the cuvette reaction cell are made automatically. The volumes used are 42 µm of reagent, 100 µm of serum 1/6 diluted in APO-diluent and 500 µm of reaction buffer. The ARRAY gives the RATE UNITS for each sample. Plotting RATE UNITS versus ASO concentration from calibrators gives the calibration curve. RATE UNITS obtained from serum samples are interpolated in the calibration curve yielding the ASO concentration.

### EXAMPLE 8

### COMPARISON OF REAGENT WITH AND WITHOUT RF

A plot of RATE UNITS versus ASO concentration was made for (1) an ASO reagent according to the invention and (2) an ASO reagent that did not contain RF. The results are shown in Figure 1.

As can be seen from the graph, the use of RF in the reagent increased the sensitivity of the system about ten-fold.

It will be appreciated that the foregoing is set forth by way of illustration and not limitation and that other embodiments within the spirit of the present invention will suggest themselves to those of ordinary skill in the art. It is intended that the present invention extend to such other embodiments also.

## Claims

1. A reagent for detecting the presence of an antibody to an antigen in a sample comprising the antigen in admixture with Rheumatoid Factor antibody.

2. The reagent according to claim 1, comprising streptolysine-O in admixture with Rheumatoid Factor antibody.

3. The reagent according to claim 2, wherein the streptolysine-O is covalently bonded to a bifunctional stabilizing agent.

4. The reagent according to claim 3, wherein the bifunctional stabilizing agent is glutaraldehyde.

5. A method for screening for antibody in a sample comprising the steps of:
(a) contacting the sample to be screened for antibody with the reagent according to claim 1, and
(b) detecting an immunoprecipitation from an immunocomplexing of antibody and antigen to produce a signal indicative of the presence of antibody in the sample.

6. A method for screening for anti-streptolysine-O in serum comprising the steps of:
(a) contacting serum to be screened for anti-streptolysine-O with a reagent according to claim 1, and
(b) detecting an immunoprecipitation from an immunocomplexing of anti-streptolysine-O and streptolysine-O to produce a signal indicative of the presence of anti-streptolysine-O in the serum.

7. The method according to claim 6, wherein the reagent contains streptolysine-O covalently bonded to a bifunctional stabilizing agent.

8. The method according to claim 7, wherein the bifunctional stabilizing agent is glutaraldehyde.
